(19) Europäisches Patentamt
European Patent Office
Office européen des brevets

(11) **EP 2 483 688 B1**

(12) **EUROPEAN PATENT SPECIFICATION**

(45) Date of publication and mention
of the grant of the patent:
**20.04.2016 Bulletin 2016/16**

(51) Int Cl.:
***G01N 21/552*** (2014.01)   ***G01N 33/543*** (2006.01)

(21) Application number: **10760058.7**

(22) Date of filing: **07.09.2010**

(86) International application number:
**PCT/IB2010/054022**

(87) International publication number:
**WO 2011/036589 (31.03.2011 Gazette 2011/13)**

(54) **METHOD FOR CHARACTERIZATION OF BIOLOGICAL BONDS**

VERFAHREN ZUR KENNZEICHNUNG BIOLOGISCHER BINDUNGEN

PROCÉDÉ DE CARACTÉRISATION DE LIAISONS BIOLOGIQUES

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **28.09.2009 EP 09171470**

(43) Date of publication of application:
**08.08.2012 Bulletin 2012/32**

(73) Proprietor: **Koninklijke Philips N.V.
5656 AE Eindhoven (NL)**

(72) Inventors:
• **VAN OMMERING, Kim**
**NL-5656 AE Eindhoven (NL)**
• **SCHLEIPEN, Johannes, J. H. B.**
**NL-5656 AE Eindhoven (NL)**
• **NIEUWENHUIS, Jeroen, H.**
**NL-5656 AE Eindhoven (NL)**

(74) Representative: **van Velzen, Maaike Mathilde
Philips Intellectual Property & Standards
High Tech Campus 5
5656 AE Eindhoven (NL)**

(56) References cited:
**US-A- 5 843 651**

• **JANSSEN X J A ET AL: "Controlled torque on superparamagnetic beads for functional biosensors", BIOSENSORS AND BIOELECTRONICS, ELSEVIER BV, NL, vol. 24, no. 7, 15 March 2009 (2009-03-15) , pages 1937-1941, XP025958957, ISSN: 0956-5663, DOI: DOI:10.1016/J.BIOS.2008.09.024 [retrieved on 2008-10-14]**

• **KIM VAN OMMERING, MARJO KOETS, RIK PAESEN, LEO J VAN IJZENDOORN AND MENNO W J PRINS: "Bond characterization by detection and manipulation of particle mobility in an optical evanescent field biosensor", JOURNAL OF PHYSICS D: APPLIED PHYSICS, vol. 43, no. 38, 385501, 7 September 2010 (2010-09-07), pages 385501-1-385501-12, XP002618601, DOI: 10.1088/0022-3727/43/38/385501**

• **VAN OMMERING, KIM; PAESEN, RIK; VAN ZON, HANS B. A.; KOETS, MARJO; VAN IJZENDOORN, LEO J.; PRINS, MENNO W. J.;: "Electrostatic height modulation of bound particle labels by buffer exchange in an evanescent wave biosensor", J. APPL. PHYS, vol. 108, no. 3, 034702, 10 August 2010 (2010-08-10), pages 034702-1-034702-5, XP002618602, ISSN: 0021-8979, DOI: 10.1063/1.3466985**

• **BRULS D M ET AL: "Rapid integrated biosensor for multiplexed immunoassays based on actuated magnetic nanoparticles", LAB ON A CHIP, ROYAL SOCIETY OF CHEMISTRY, CAMBRIDGE, GB, vol. 9, no. 24, 1 January 2009 (2009-01-01), pages 3504-3510, XP002583698, ISSN: 1473-0197, DOI: DOI:10.1039/B913960E [retrieved on 2009-10-15]**

EP 2 483 688 B1

**(Cont. next page)**

- **KIM VAN OMMERING1, JEROEN H. NIEUWENHUIS, LEO J. VAN IJZENDOORN, BERT KOOPMANS, AND MENNO W. J. PRINS: "Confined Brownian motion of individual magnetic nanoparticles on a chip: Characterization of magnetic susceptibility", APPLIED PHYSICS LETTERS, vol. 89, no. 14, 142511, 6 October 2006 (2006-10-06), pages 142511-1-142511-3, XP002618603, DOI: 10.1063/1.2360246**
- **KIM VAN OMMERING, CAROLIEN C. H. LAMERS, JEROEN H. NIEUWENHUIS, LEO J. VAN IJZENDOORN, AND MENNO W. J. PRINS: "Analysis of individual magnetic particle motion near a chip surface", JOURNAL OF APPLIED PHYSICS, vol. 105, no. 10, 104905, 21 May 2009 (2009-05-21), pages 104905-1-104905-10, XP002618604, DOI: 10.1063/1.3118500**
- **WELLMAN A D ET AL: "Multiplexed, Waveguide Approach to Magnetically Assisted Transport Evanescent Field Fluoroassays", ANALYTICAL CHEMISTRY, AMERICAN CHEMICAL SOCIETY, US, vol. 79, no. 17, 1 September 2007 (2007-09-01), pages 6622-6628, XP002558341, ISSN: 0003-2700, DOI: DOI:10.1021/AC070839Y [retrieved on 2007-08-02]**

**Description**

FIELD OF THE INVENTION

The invention relates to the field of intensity measurements of a light scattering label bound to a surface of a support using an optical evanescent field.

BACKGROUND OF THE INVENTION

Total internal reflection ("TIR") or total internal reflection microscopy ("TIRM") is well known in the art. If light strikes an interface between a first medium and a second medium below a critical angle, the latter having a lower refractive index than the former, the light is totally reflected within the first medium. Under these conditions, an electromagnetic field, the "evanescent field", is generated. The evanescent field penetrates into the rarer medium. Its energy dissipates exponentially as a function of distance from the interface. A parameter, the "penetration depth" is defined as the distance from the interface at which the energy of the evanescent field has fallen to 1/e times the energy value at the interface.

The optical evanescent field allows the optical detection of objects within the penetration depth. The value of the penetration depth is in the order of 100nm, depending on the angle of incidence, wavelength of the light and the refractive indices of the first and second medium.

Known methods for detecting objects within an evanescent field are using fluorescent objects, which can be excited by the field. The fluorescence of the excited objects is measured.

Also light scattering objects can be used as detection objects. Within the evanescent field, these objects scatter light generated by the evanescent field. The scattered light can be monitored with a camera. US-Patent No. 5 599 668 discloses a method for measuring in real time the binding or the melting of a light scattering label, i.e., a detectable object, on multiple capture sites on a support comprising a DNA array. According to this method, the intensity of light scattered on such labels is measured within the depth of an evanescent optical field.

SUMMARY OF THE INVENTION

It is an object of the invention to provide a method for characterization of biological bonds. A further object of the invention is to provide an application of this method.

This object is achieved by a method according to claim 1.

Bond characteristics are, for example, the length of the bond, the flexibility of the bond and the mobility of the bond. Measuring the fluctuations in the intensity of the scattered light over time while the label is bound to the surface and analyzing the measured intensity values allows to draw conclusions on said parameters and, therefore, on the type of bond. The method according to the invention allows to identify different bonds and/or to distinguish between different bonds.

In one application of the invention, the method is used for distinguishing between specific and non-specific bonds.

In another application of the invention, the method is used for distinguishing between a label bound to the surface of the support by a single bond and a label bound to the surface of the support by multiple bonds.

Both applications can be advantageously used in a biosensor, where non-specific bonds and/or multiple bonds in an assay normally limit the sensitivity of the biosensor. The method according to the invention allows to distinguish between specific, non-specific and multiple bonds, which allows to improve the sensitivity of the biosensor.

Furthermore, the method can be used for distinguishing between two different specific bonds and/or for measuring bond length and/or bond flexibility of the at least one bond.

It is preferred that the time frame for measuring the scattered intensity of the label over time is $\geq 1$ second, preferably $\geq 20$ seconds. Such a time frame allows the label to take nearly all positions being at its disposal, especially its minimum height and its maximum height relative to the surface of the support.

Moreover, it is preferred that each intensity measurement is done with an exposure time of $\leq 40$ ms, preferably $\leq 1$ ms. Such an exposure time allows to measure the exact position of the label and not an averaged position due to movement within the exposure time.

According to a preferred embodiment of the invention the intensity of the scattered light is being measured space-resolved to account for different xy-positions of the label in the in-plane of the surface of the support. Space resolved measuring of the scattered intensity over time gives extra information which can be used for characterizing the bond. A spatial resolution of $\leq 75$ nm, preferably of $\leq 15$ nm is preferred.

According to another preferred embodiment of the invention, in step b) the at least one label is temporary pulled towards the surface and/or away from the surface and/or into different directions parallel to the surface. Pulling the label into different directions and measuring the scattered intensity under these conditions can give further information about the bond. Pulling the label into different directions can be achieved, if the label is, for example, magnetic, with a magnet. Other kinds of actuations are also possible.

According to another preferred embodiment of the invention, the label can be a uniform or a non-uniform particle.

A non-uniform label can, due to its non-uniformity, lead to further intensity fluctuations, for example when the bound particle tilts or rotates around its center. Therefore, in case of non-uniform labels, not only the height but further parameters influence the intensity. The degree of influence also depends on the type of bond. This dependence can be used to obtain further information and, therefore, to increase detection sensitivity. Furthermore, also for non-uniform labels external forces can be used to pull the label in different directions to obtain further information about the bond.

According to another preferred embodiment of the invention, the bond mobility of the bond is determined by determining the ratio between the maximum and the minimum intensity in the time frame, by determining the standard deviation of the measured intensity values, by making a histogram of the measured intensity values and analyzing its shape, and/or by analyzing average diffusion coefficients or the power spectrum by Fourier analysis.

According to a preferred embodiment the bond comprises one or more biomolecules.

As used herein the term "biomolecule" refers to a structure, constructed in such way that it permits binding to a support as well as to a target, to facilitate formation of the bond. Examples for such biomolecules are nucleic acids, oligonucleotides, amino acids, monosaccharide, proteins, polysaccharides and haptens, e.g. antibodies or lectins, which can bind molecular structures such as DNA sequences, antibodies or antigens, or enzymes. Furthermore the term also refers to the common linking agents as they are used in affinity chromatography, immohistochemistry and in protein-tags, examples of such agents include ligand - receptor interactions, biotin and streptavidin (avidin) and the GST-tag.

In an especially preferred embodiment the bond comprises a capture entity - here an antibody bound to a surface - a target molecule and a linker. Hence the antibody binds the target molecule and the linker attaches the label to the target molecule, thereby connecting the label via the formed bond to the surface.

Especially, the linker could be part of a prepared assay. A label plus linker can catch a target molecule within a fluid and bring it to the cartridge surface binding the target molecule to the antibody on the cartridge surface.

BRIEF DESCRIPTION OF THE DRAWINGS

These and other aspects of the invention will be apparent from and elucidated with reference to the embodiments described hereinafter.

In the drawings:

Fig. 1    is a schematic view of a setup for generating an optical evanescent field and for measuring the fluctuations in intensity of scattered light of an object in the evanescent field;

Fig. 2    is a schematic view of a label bound to a surface of a support in three different ways, first, by a single bond, second, without a bond, and third, with a multiple bond;

Fig. 3    is a schematic view of a specifically bound label without exerting a force on it and with exerting a force on it, and of a non-specifically bound label without exerting a force on it and with exerting a force on it;

Fig. 4    is a graph displaying the scattered light intensity over time measured on a specifically bound label;

Fig. 5    is a graph displaying the scattered light intensity over time measured on a non-specifically bound label;

Fig. 6    is a graph displaying the scattered light intensity over time measured on a bound label while temporarily applying a magnetic field;

Fig. 7    shows three graphs displaying the x- and y-position of the label and the scattered light intensity over time measured on a specifically bound label while temporarily applying a magnetic field;

Fig. 8    is a graph displaying the scattered light intensity over time measured on a label bound to a surface of support by a bond comprising DNA;

Fig. 9    is a graph displaying the maximum ratio of the measured intensity versus DNA length of several intensity measurements on a series of identical labels bound with DNA of different lengths to a surface of a support; and

Figs. 10-13    show several xy-plots of the maximum height and the averaged height of four examples of labels shown in Fig. 9.

DETAILED DESCRIPTION OF EMBODIMENTS

Variations in the average height of a label bound to a surface of a support and variations in mobility of the height of labels with different bonds are expected to be in the order of 1-100 nm and, therefore, are very small. If these types of displacements were for example to be detected microscopically, it is advantageous to have a high optical resolution. Moreover, in assay applications, for example for a biosensor, particularly at the lower concentrations where the sensitivity of the biosensor is challenged, it is advantageous to observe a fairly large surface area (typically > 0.1 mm$^2$) to have a sufficient number of labels to get acceptable statistics.

The penetration depth of an evanescent field is typically in the range of 20-200 nm, which makes it very suitable for measuring small displacements. A label moving more than the penetration depth away from the surface (z-direction) would go from maximum scattered light to almost no scattered light, which can be detected very precisely using a camera. For example, with a common CCD camera (255 grey values) and a penetration depth of around 100 nm, a resolution below a few nanometers can reasonably be expected.

Moreover, the requirements on in-plane optical resolution (*x-y*) are not influenced. When using lower concentrations, as in sensitive biosensor measurements, the label surface density is typically quite small, e.g. 1 label / 100 $\mu$m$^2$, which is equivalent to an area of 10 $\mu$m x 10 $\mu$m. Only one pixel per label is needed, so the required in-plane resolution of the imaging system only needs to be 5 $\mu$m, and yet vertical displacements of less than 100 nm can be detected.

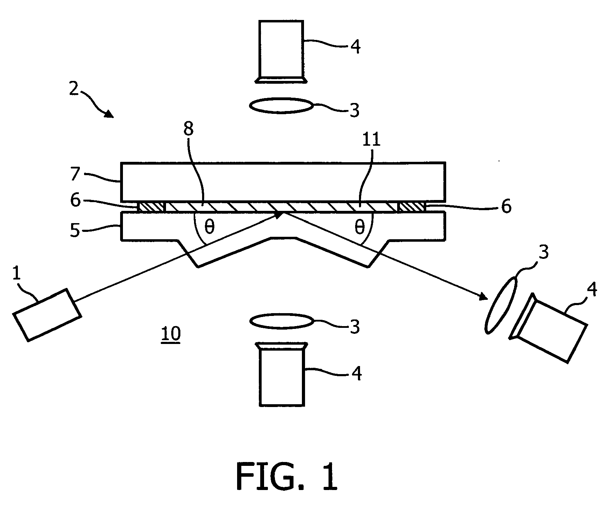

Fig. 1 shows a schematic view of a setup for generating an optical evanescent field and for measuring the intensity of scattered light of a label in the evanescent field.

The setup comprises a light source 1, a cartridge 2, an optical system 3 and a camera 4.

The cartridge 2 comprises a support 5, two spacers 6 and a cover plate 7. The support 5, the spacers 6 and the cover plate 7 are forming a cavity 8, which is filled with a fluid. The support 5 and cover plate 7 are transparent for the wavelength of the light emitted by light source 1.

The refractive index of the support 5 is higher than the refractive index of the fluid inside the cavity 8. The light 10 being emitted by the light source 1 is coupled into the cartridge 5 and directed on the interface 11 between the fluid and the support 5. The angle of incidence θ of the light 10 striking the interface 11 is smaller than the critical angle, thus the light 10 is totally reflected on the interface 11, generating an optical evanescent field which penetrates the fluid inside the cavity 8.

Light scattering labels inside the fluid within the range of the evanescent field will scatter the light of the evanescent field. The intensity of the scattered light depends on the distance of the label to the interface 11.

The scattered light can be detected with the camera 4. The optical system 3, here symbolically displayed as a simple lens, focuses the scattered light on the camera 4. The optical system 3 can comprise a plurality of optical components.

Different arrangements of the optical system 3 and the camera 4 are possible for measuring the scattered light. For example, the optical system 3 and the camera 4 can be positioned above the cartridge 2 or below the cartridge 2. In this case, the signal intensity detected by the camera 4 would go from maximum brightness to almost completely darkness for a perpendicular displacement of a light scattering label away from the surface. Furthermore, the optical system 3 and the camera 4 can be positioned in the light path of the reflected light 10. The signal intensity would go from reduced to maximum brightness for a perpendicular displacement of a label from the surface.

The penetration depth of the evanescent field, defined as

$$p_d = \frac{\lambda}{2\pi\sqrt{n_1^2 \sin^2\theta - n_2^2}},$$

with $n_1$ the refractive index of the support 5 and $n_2$ the refractive index of the fluid, can be tuned by changing the wavelength of the light λ or the angle of incidence θ of the light beam 10. This allows to choose between a wide range of displacement detection with limited displacement resolution (high penetration depth), and a short range of displacement detection with high displacement resolution (low penetration depth). By using for example two different wavelengths or two different angles of incidence and filtering at the detection optics, one may combine both regimes.

The setup as described can be used to carry out the methods as described in the following passages. However, alternative setups as known in the art also might be usable for this purpose.

The light intensity of light scattered on a label depends on the distance of the label to the surface of the support. If a label is bound to the surface by a bond, the distance of the bound label to the surface depends on the type of bond. Therefore, the intensity of the scattered light allows to draw a conclusion on the type of bond.

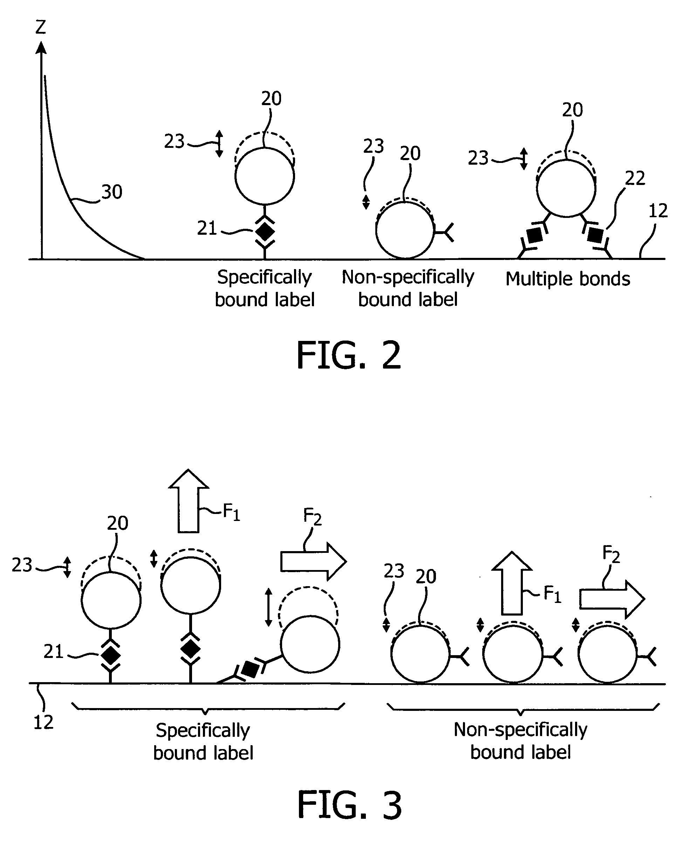

Fig. 2 is a schematic view of a label 20 bound to a surface 12 of the support 5 in three different ways, first, by a single bond 21, second, without a bond, and third, with a multiple bond 22. The decreasing intensity of the evanescent field in a direction perpendicular to the surface 12 (z-direction) is shown with graph 30.

The scattered light intensity of a label 20 in an evanescent field will depend on the distance of the label 20 to the surface. As can been seen from Fig. 2, the height of the label 20 will vary for different types of bonds, for example specific (single bond 21) vs. non-specific (no bond) or single bonds 21 vs. multiple bonds 22. For the specifically bound label 20, a "normal" height is to be expected, for the non-specifically bound label 20 (no bond) and the label 20 bound by multiple bonds 22 a reduced height is to be expected.

Also, the degree of mobility in the height of the label 20 will vary for different bonds. It is to be expected that the mobility of a label 20 bound with a single bond 21 to the surface 12 is higher than a label 20 bound non-specifically to the surface 12, or a label 20 bound with a multiple bond 22 to the surface 12. The thermal vertical mobility due to Brownian motion is displayed with arrows 23.

For uniform labels, like polystyrene beads, it is known that the scattered light intensity decreases for an increasing distance between label and surface with the same exponential function as the evanescent field, except for minor deviations nearby the surface. However, also other factors may contribute to the scattered light intensity of a label such as absorption, scattering inhomogeneities, particle roughness and rotational diffusion, possibly leading to different relations between the height of the label and scattered intensity.

Fig. 3 is a schematic view of a specifically bound label 20 without applying a force on it and with applying a force on it, and of a non-specifically bound label 20 without applying a force on it and with applying a force on it.

The height of the label 20 and its mobility in height can also be influenced by external forces, such as fluidic forces or electrostatic forces. This adds an extra dimension to distinguishing between different bond types, as the degree of response to the force will also vary for different bond types, as shown in Fig. 3.

In case of a specifically bound label 20, if an upward force $F_1$ is exerted on the label 20, the bond 21 is stretched, increasing the height and reducing the mobility of the label 20. If a sideward force $F_2$ is exerted, the label 20 is pulled to the surface 12, reducing the height and increasing the mobility of the label 20. In case of a non-specifically bound label 20, if an upward force $F_1$ or a sideward force $F_2$ is exerted on the label 20, only little change in the height and the mobility of the label is to be expected.

When magnetic labels are used, the application of an external force is very easy and controllable as this can be done magnetically. This allows for example force modulation for better statistics.

In order to obtain a good displacement resolution, also a good time resolution is needed. If the imaging system is able to detect a few nanometers displacement of a label, the time of imaging (exposure time) should be chosen such that a label does not move more than a few nanometers.

Typical values for free diffusion of 500 nm labels in a solution are 1 $\mu$s to diffuse 1 nm, or 1 ms to diffuse 30 nm. However, nearby a surface the hydrodynamic drag can decrease diffusion down to a factor of 10. A 500 nm particle could then move 5 nm in 1 ms. Finally, a bound label is inhibited by the bond in moving, which will decrease its typical 'speed' even more, relaxing requirements on exposure times. An estimated preferred exposure time for obtaining 1 nm resolution for 500 nm particles is a maximum exposure time of 1 ms.

When choosing larger exposure times than typical diffusion times, fluctuations in intensity by particle movement will gradually be averaged out, and the extremes of the intensity distributions will become distorted. However, when comparing intensity signals of labels measured with equal exposure times, there will still be information in these distributions.

Finally, it should be noted that even when choosing small exposure times, it is not necessary to use advanced high frame rate cameras, as Brownian motion can be sampled stroboscopically, for example with 30 frames per second with 1 ms exposure time per frame.

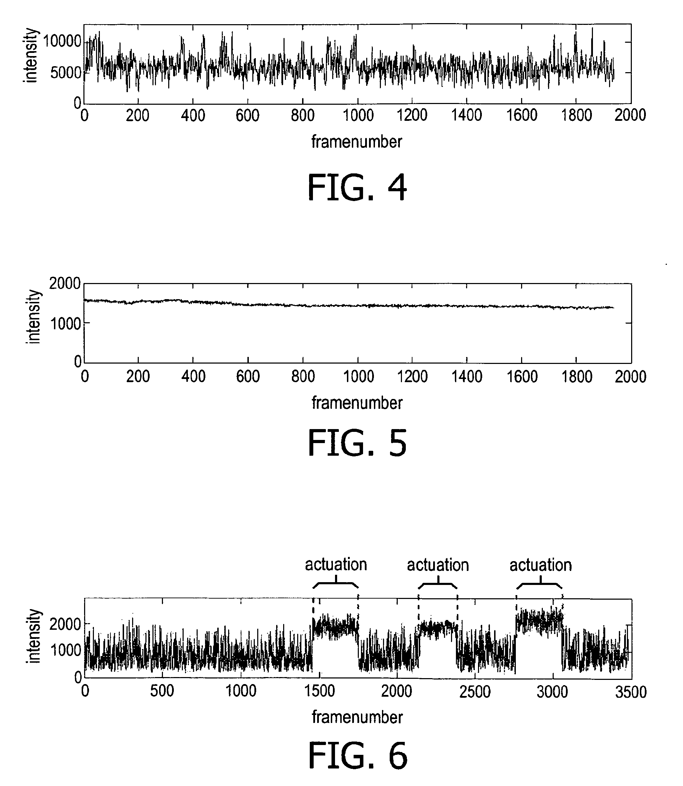

Figs. 4 to 7 show different graphs displaying the scattered light intensity over time measured on a specifically bound label (Fig. 4, 6 and 7) and on a non-specifically bound label (Fig. 5).

For these measurements, the bottom part of a typical polystyrene standard cartridge was used as a support 5, see Fig. 1, with a cover plate 7 made from glass for better visibility. An electromagnet (not shown in Fig. 1) is mounted below this cartridge 2 to pull labels towards the surface 12 of the support 5. The scattered light from labels is detected by a microscope 3 and CCD-camera 4 from the top. The light source 1 is a 50 mW laser with a wavelength of 658 nm, focused on a spot of approximately 200 x 200 $\mu$m of the interface 11 to obtain high light intensity for measurements with low exposure time ($\sim$ 1 ms) to accurately measure fluctuations of the labels due to Brownian motion.

In this embodiment, the target is a double stranded DNA molecule with a length of 99 nm, which is bound to TexasRed on one end and to biotin on the other end. Moreover in this embodiment the light scattering label is a superparamagnetic bead with a diameter of about 500nm, which is coated with a linker molecule, here streptavidin. Thus the TexasRed-bound end of the DNA will bind to the anti-TexasRed antibody on the surface 12, while the biotin-bound end of the DNA will bind to streptavidin coating of the superparamagnetic bead. In this way the antibody, the target and the linker are forming a bond, which connects the label to the surface. In this case, the superparamagnetic bead is a non-uniform particle composed of thousands of 5-10 nm iron oxide grains inside a polystyrene matrix. Due to the small grain size the iron oxide becomes superparamagnetic, so without a magnetic field the particle does not have a magnetic moment, and in a magnetic field a magnetic moment is induced in the particle.

In a next step, the intensity of scattered light of one or more of such labels is measured over time while the label is bound to the surface 12 and while the label is within an optical evanescent field.

Fig. 4 shows a graph of the scattered light intensity as function of frame number for one label bound to the surface with a bond comprising a 99 nm DNA. In this embodiment, the frame number is 120 frames per second (fps), the exposure time is 0.96 ms. The penetration depth of the evanescent field is 90nm.

Fig. 4 shows a typical intensity dataset for a bound label. Fluctuations in intensity can clearly be observed and can be up to a factor 5. If the scattered intensity would be exponentially decaying with the bead height, one calculates a height variation of 145 nm, which is unlikely for DNA of 99 nm. Apparently, also other factors influence the scattering intensity, like bead inhomogeneities. From SEM (scanning electron microscope) pictures it is known, that such labels are quite rough.

Fig. 5 shows a graph of the scattered light intensity as function of frame number (120 fps, exposure time 0.96 ms) for one label that was non-specifically (no bond) bound to the surface.

In this case, the fluctuations in intensity are at maximum 14%, which is clearly different from beads bound with DNA (>factor 10 difference), as shown in Fig. 4. Furthermore, this bead showed no movement in-plane (xy-position) within the lateral resolution in this imaging system (15 nm). Other beads bound with 99 nm DNA showed lateral movements between 30-200 nm.

It should be noted that the averaged scattered intensities of different 500 nm beads are varying orders of magnitudes between beads due to their size distribution.

Fig. 6 shows a graph of the scattered light intensity as function of frame number (120 fps, exposure time 0.96 ms) for one label that was specifically bound to the surface. The label is temporary pulled to the surface 12 by exerting a force on the label, in this case by applying a magnetic field.

As is shown in Fig. 6, the average intensity increases upon magnetic actuation as the beads are pulled towards the surface. The fluctuations in intensity decrease, as the beads are magnetically confined in their height.

Alternatively or in addition, the force exerted on the label can have a different direction, for example a sideward or an upward direction.

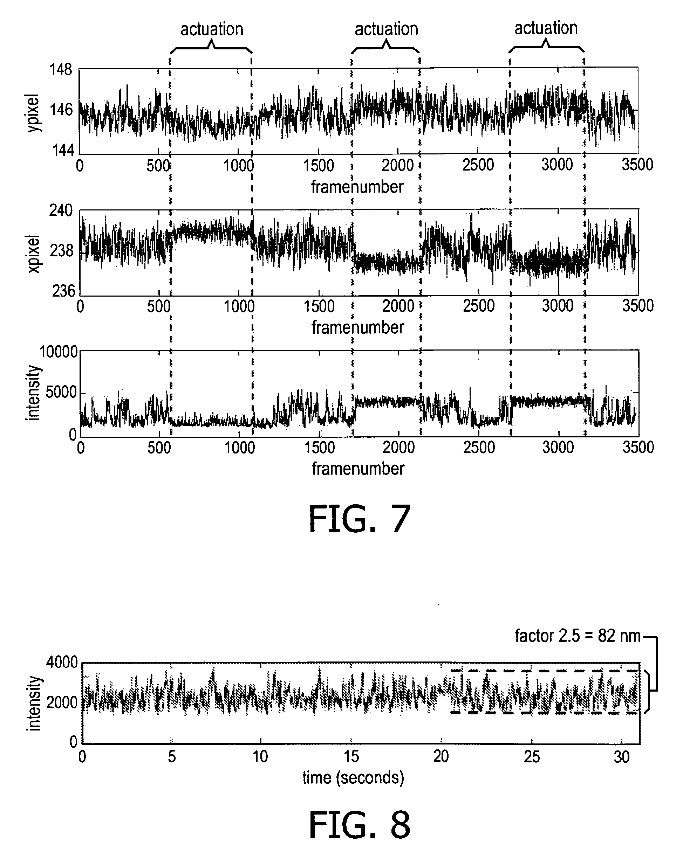

Fig. 7 shows three graphs displaying the x- and y-position of the label and the scattered light intensity over time measured on a specifically bound label while temporarily applying a magnetic field. The spatial resolution is 1 pixel / 150 nm with a sub-pixel resolution of 15nm.

As can be seen in Fig. 7, the bound bead shows both a decreasing average intensity upon magnetic actuation and an increasing average intensity, depending on the position of "landing" on the surface. This indicates that bead inhomogeneities play a role. Independent of the behavior of the average intensity, the fluctuations in intensity always decreased upon magnetic actuation.

Fig. 8 is a graph displaying the scattered light intensity over time of another measurement on a label bound to a surface of a support by a bond.

The method for measuring the intensity of the scattered light of the bead is in principal the same as described above. However, in this embodiment, the label is a uniform polystyrene bead. The bond, which attaches the bead to the surface 12, comprises a DNA with a length of 99nm. If it is assumed that the scattered intensity decreases exponentially with the bead height, one calculates a height variation of 82 nm of the bead.

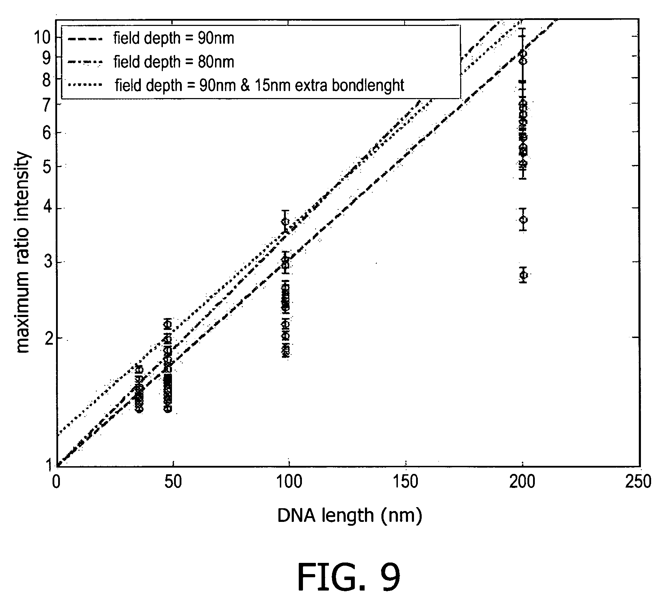

Fig. 9 shows a graph displaying the maximum ratio of the measured intensity versus DNA length of several intensity measurements on a series of identical labels bound to a surface 12 of a support 5 by a bond. Each label is a uniform polystyrene bead. Each bond comprises a DNA with a certain lengthIn this case, four different lengths of the DNA are used, namely 36nm, 48nm, 99nm and 201nm. Fig. 9 thus shows 4 distinguishable datasets of in each case 11-17 measured labels. The maximum ratio of the intensity is determined by the maxima and the minima of the intensity fluctuations. The measurements have been carried out as described above.

As shown in Fig. 9, for polystyrene beads the maximum intensity fluctuations that are measured are indeed at maximum at the values expected from the evanescent field depth under the condition of a field depth of 90 nm, which is also the calculated value, and an additional bond length of 15 nm. The additional bond length might be caused due to the antibodies and linker-molecules.

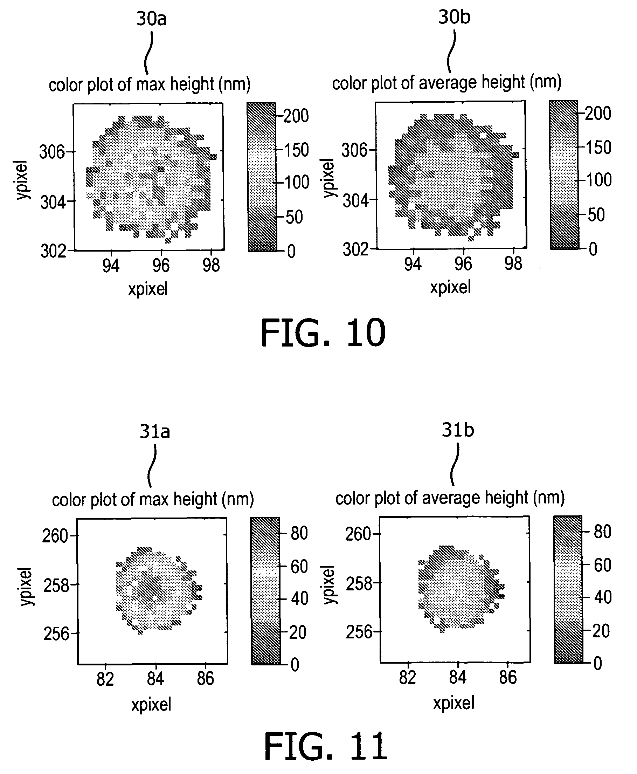

Figs. 10 to 13 show several xy-plots of the maximum height and the averaged height of the four labels of Fig. 9. One x- or y-pixel equals a distance of 150 nm with a sub-pixel resolution of 15nm.

In Fig. 10, Plot 30a displays the maximum height determined from intensity measurements on the label with a DNA-length of 201 nm in dependence on the x- and y position of the label, plot 30b displays the averaged height determined from intensity measurements on the label with a DNA-length of 201 nm in dependence on the x- and y position of the label. The maximum height is determined by taking a certain square of x,y positions (for example of 0.2x0.2 pixel), then selecting all data points whose positions fall within this square, and finally determining the maximum height within this selected ensemble. The averaged height is determined by taking the same square of x,y positions and the same data point selection, and then determining the average height of the data points within this selected ensemble.

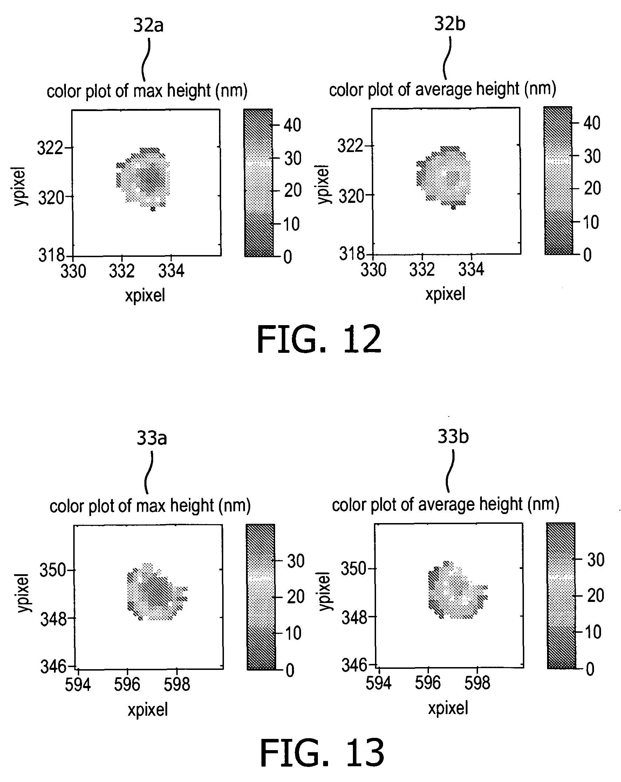

Similar, plot 31a and 31b shown in Fig. 11 display the xy-dependent maximum height and the averaged height, respectively, determined from measurements on the label with a DNA-length of 99 nm, plot 32a and 32b shown in Fig. 12 display the xy-dependent maximum height and the averaged height, respectively, determined from measurements on the label with a DNA-length of 48 nm, and plot 33a and 33b shown in Fig. 13 display the xy-dependent maximum height and the averaged height, respectively, determined from measurements on the label with a DNA-length of 36 nm.

One fact that can be seen in the plots shown in Fig. 10 is that the mobility of the label is more and more restricted, i.e. the maximum height displacement decreases, when reducing the length of the DNA, i.e., the bond.

Another fact that can be seen from Fig. 10 is that, as the DNA length decreases, the average height starts to resemble the maximum height more closely, which means the molecule is stiffer and, therefore, spends more time in a stretched position.

As shown in the measurements, bonds with different characteristics lead to different measurement results. Therefore, the method according to the invention allows to characterize a bond or to distinguish between different bonds.

When the intensity signal vs. time is measured for single labels, one can calculate the maximum and minimum intensity in a certain time frame (as done in Figure 4 and 5). The ratio between these two intensity values gives information on the degree of mobility of the label and thus on the type on bond.

Another possibility is to determine the standard deviation of the measured intensity signal, which gives the average degree of fluctuations in mobility and is thus also characteristic for the type on bond.

Also, a histogram of the measured intensity values can be made. This gives both the opportunity to remove rare events or deviating values due to noise, and the opportunity to analyze the histogram shape. The shape of the histogram will be dependent on the density of states of the label in the position space (the amount of time the label is furthest away from the surface will be different than the amount of time the label is closer to the surface), and will therefore also give information on the type of bond.

When magnetic actuation is used to pull the bead towards or away from the surface, see Fig. 6 and 7, the same analysis can be applied to the intensity data in the actuated state (for example analyzing the ratio maximum/minimum intensity, analyzing the standard deviation and analyzing the histogram) and all values can be compared to the non-actuated state, leading to extra information on the type of bond.

With high position resolution in combination with single label intensity measurements, both position and intensity data can also be combined, as done in Fig. 10. Parameters such as the maximum intensity as function of the position, the average intensity as function of the position and the standard deviation as function of position give additional information on the density of states of the label in the position space, and therefore on the type of bond, especially for example on bond length and bond flexibility.

One can also use the time dynamics information in the measured intensity signal to analyze the kinetics of the system, for example by analyzing average diffusion coefficients or the power spectrum by Fourier analysis, giving additional information on bond mobility.

Finally, when not only particle height but also particle properties (shape, roughness) are involved in the measured intensity signal (which is the case for certain types of magnetic particles as we have shown), this can be used to obtain more information. Fluctuations that are higher than to be expected if only considering the height of the label can actually increase detection sensitivity. Smart actuation schemes can be developed, such as pulling the bound bead to different directions, and analyzing the different states it samples. More bond mobility will lead to more variety in possible sampled states, and thus the different measured signals are a measure for bond mobility. The method according to the invention can be applied in numerous applications.

In one embodiment, the method is used in a biosensor for distinguishing between specific and non-specific bonds, thus enhancing the detection sensitivity and improving the detection limit of the biosensor.

In another embodiment the method is used to detect the presence of single or multiple bonds, giving a better understanding of the relation between target concentration and detected number of labels.

Finally, the invention is used for functional biosensors that can not only detect the presence of target molecules, but can also give information on the molecule, for example bond length or bond flexibility or changes in state of the molecule. For example, the method can be used to obtain information on persistence length of DNA, which is influenced by external circumstances such as salt concentration, to

detect the folding/unfolding of proteins, and/or to detect enzyme reactions on the bound target molecule that change the state of the target molecule.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. Any reference signs in the claims should not be construed as limiting the scope.

**Claims**

1. A method for the characterization of biological bonds, comprising the steps:

   a) Providing an assay comprising at least one light scattering label bound to a surface of a support by at least one bond;
   b) Measuring the fluctuations in the intensity of scattered light of the label in an optical evanescent field over time while the label is bound to the surface whereby the intensity of the scattered light is measured space-resolved to account for different xy-positions of the label in-plane of the surface.

2. The method according to claim 1, whereby the time frame for measuring the scattered intensity of the label over time is $\geq$ 1 second, preferably $\geq$ 20 seconds.

3. The method according to claim 1 or 2 whereby the exposure time for each intensity measurement is $\leq$ 40 ms, preferably $\leq$ 1 ms

4. The method according to claim 1, whereby a spatial resolution of $\leq$ 75 nm, preferably of $\leq$ 15 nm is used.

5. The method according to one of the claims 1 to 4, whereby in step b) the at least one label is temporary pulled towards the surface and/or away from the surface and/or into different directions parallel to the surface.

6. The method according to one of the claims 1 to 5, whereby the bond mobility of the bond is determined by determining the ratio between the maximum and the minimum intensity in the time frame, by determining the standard deviation of the measured intensity values, by making a histogram of the measured intensity values and analyzing its shape, and/or by analyzing average diffusion coefficients or the power spectrum by Fourier analysis.

7. The method according to one of the claims 1 to 6, whereby the label is a uniform particle or a non non-uniform particle.

8. The method according to one of the claims 1 to 7, whereby the bond comprises one or more biomolecules.

9. The method according to one of the claims 1 to 8, whereby the bond comprises a catching antibody, a target molecule and a linker molecule, the antibody attaching the target molecule to the surface of the support, the linker molecule attaching the label to the target molecule.

10. Method according to one of the claims 1 to 9, used for distinguishing between specific and non-specific bonds.

11. Method according to one of the claims 1 to 9, used for distinguishing between a label bound to the surface of the support by a single bond and a label bound to the surface of the support by a multiple bond.

12. Method according to one of the claims 1 to 9, used for measuring bond length and/or bond flexibility of at least one bond.

**Patentansprüche**

1. Verfahren zur Charakterisierung biologischer Bindungen, das die folgenden Schritte umfasst, wonach:

a) ein Assay mit mindestens einem Licht streuenden Marker vorgesehen wird, der durch mindestens eine Bindung an eine Oberfläche eines Trägers gebunden ist;
b) die Fluktuationen in der Intensität von Streulicht des Markers in einem optischen, evaneszenten Feld im Zeitablauf gemessen werden, während der Marker an die Oberfläche gebunden ist, wobei die Intensität des Streulichts ortsaufgelöst gemessen wird, um verschiedene xy-Positionen des Markers in der Ebene der Oberfläche zu erfassen.

2. Verfahren nach Anspruch 1, wobei der Zeitrahmen zur Messung der Streuintensität des Markers zeitlich $\geq 1$ Sekunde, vorzugsweise $\geq 20$ Sekunden, ist.

3. Verfahren nach Anspruch 1 oder 2, wobei die Belichtungszeit für jede Intensitätsmessung $\leq 40$ ms, vorzugsweise $\leq 1$ ms, ist.

4. Verfahren nach Anspruch 1, wobei eine räumliche Auflösung von $\leq 75$ nm, vorzugsweise von $\leq 15$ nm, verwendet wird.

5. Verfahren nach einem der Ansprüche 1 bis 4, wobei in Schritt b) der mindestens eine Marker temporär zu der Oberfläche hin und/oder von der Oberfläche weg und/oder in verschiedene Richtungen parallel zu der Oberfläche gezogen wird.

6. Verfahren nach einem der Ansprüche 1 bis 5, wobei die Bindungsmobilität der Bindung durch Ermitteln des Verhältnisses zwischen der maximalen und der minimalen Intensität in dem Zeitrahmen, durch Ermitteln der Standardabweichung der gemessenen Intensitätswerte, durch Erstellen eines Histogramms der gemessenen Intensitätswerte und Analysieren seiner Form, und/oder durch Analysieren von mittleren Diffusionskoeffizienten oder des Leistungsspektrums durch Fourier-Analyse ermittelt wird.

7. Verfahren nach einem der Ansprüche 1 bis 6, wobei der Marker ein uniformes Teilchen oder ein nicht-uniformes Teilchen ist.

8. Verfahren nach einem der Ansprüche 1 bis 7, wobei die Bindung ein oder mehrere Biomoleküle umfasst.

9. Verfahren nach einem der Ansprüche 1 bis 8, wobei die Bindung einen Fangantikörper, ein Zielmolekül und ein Linker-Molekül umfasst, wobei der Antikörper das Zielmolekül an die Oberfläche des Trägers anlagert, wobei das Linker-Molekül den Marker an das Zielmolekül anlagert.

10. Verfahren nach einem der Ansprüche 1 bis 9, das angewandt wird, um zwischen spezifischen und nicht-spezifischen Bindungen zu unterscheiden.

11. Verfahren nach einem der Ansprüche 1 bis 9, das angewandt wird, um zwischen einem durch eine einzelne Bindung an die Oberfläche des Trägers gebundenen Marker und einem durch eine Mehrfachbindung an die Oberfläche des Trägers gebundenen Marker zu unterscheiden.

12. Verfahren nach einem der Ansprüche 1 bis 9, das angewandt wird, um Bindungslänge und/oder Bindungsflexibilität von mindestens einer Bindung zu messen.

## Revendications

1. Méthode pour la caractérisation de liaisons biologiques, comprenant les étapes de :

   a) utilisation d'une analyse comprenant au moins un marqueur de dispersion de lumière lié à une surface d'un support par au moins une liaison ;
   b) mesure des fluctuations de l'intensité de lumière dispersée du marqueur dans un champ évanescent optique au fil du temps alors que le marqueur est lié à la surface moyennant quoi l'intensité de la lumière dispersée est mesurée en résolution spatiale pour représenter différentes positions xy du marqueur dans le plan de la surface.

2. Méthode selon la revendication 1, moyennant quoi la trame temporelle pour mesurer l'intensité dispersée du marqueur au fil du temps est $\geq$ 1 seconde, de préférence $\geq$ 20 secondes.

3. Méthode selon la revendication 1 ou 2, moyennant quoi le temps d'exposition pour chaque mesure d'intensité est $\leq$ 40 ms, de préférence $\leq$ 1 ms.

4. Méthode selon la revendication 1, moyennant quoi une résolution spatiale de $\leq$ 75 nm, de préférence de $\leq$ 15 nm est utilisée.

5. Méthode selon l'une des revendications 1 à 4, moyennant quoi à l'étape b) l'au moins un marqueur est temporairement tiré vers la surface et/ou loin de la surface et/ou dans différentes directions parallèles à la surface.

6. Méthode selon l'une des revendications 1 à 5, moyennant quoi la mobilité de liaison de la liaison est déterminée en déterminant le rapport entre l'intensité maximale et l'intensité minimale dans la trame temporelle, en déterminant l'écart standard des valeurs d'intensité mesurées, en créant un histogramme des valeurs d'intensité mesurées et en analysant sa forme, et/ou en analysant les coefficients de diffusion moyens ou le spectre de puissance par une analyse de Fourier.

7. Méthode selon l'une des revendications 1 à 6, moyennant quoi le marqueur est une particule uniforme ou une particule non uniforme.

8. Méthode selon l'une des revendications 1 à 7, moyennant quoi la liaison comprend une ou plusieurs biomolécules.

9. Méthode selon l'une quelconque des revendications 1 à 8, moyennant quoi la liaison comprend un anticorps capteur, une molécule cible et une molécule lieuse, l'anticorps attachant la molécule cible à la surface du support, la molécule lieuse attachant le marqueur à la molécule cible.

10. Méthode selon l'une quelconque des revendications 1 à 9, utilisée pour faire la distinction entre liaisons spécifiques et liaisons non spécifiques.

11. Méthode selon l'une quelconque des revendications 1 à 9, utilisée pour faire la distinction entre un marqueur lié à la surface du support par une simple liaison et un marqueur lié à la surface du support par une liaison multiple.

12. Méthode selon l'une quelconque des revendications 1 à 9, utilisée pour mesurer la longueur de liaison et/ou la flexibilité de liaison d'au moins une liaison.

# FIG. 1

FIG. 2

FIG. 3

FIG. 4

FIG. 5

FIG. 6

**FIG. 7**

**FIG. 8**

FIG. 9

30a

color plot of max height (nm)

FIG. 10

30b

color plot of average height (nm)

31a

color plot of max height (nm)

FIG. 11

31b

color plot of average height (nm)

FIG. 12

FIG. 13

**EP 2 483 688 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5599668 A